(19) Europäisches Patentamt
European Patent Office
Office européen des brevets

(11) Numéro de publication: **0 092 627**
**B1**

(12)
# FASCICULE DE BREVET EUROPEEN

(45) Date de publication du fascicule du brevet: **29.05.85**

(21) Numéro de dépôt: **82401218.1**

(22) Date de dépôt: **30.06.82**

(51) Int. Cl.⁴: **C 07 C 49/84**, C 07 C 45/00, C 07 C 45/43, C 07 C 79/36, C 07 C 149/36, C 07 C 149/40

(54) **Procédé de préparation de di ou trifluorométhoxyphénylcétones ou de di ou trifluorométhylthiophénylcétones.**

(30) Priorité: **22.04.82 FR 8206903**

(43) Date de publication de la demande: **02.11.83 Bulletin 83/44**

(45) Mention de la délivrance du brevet: **29.05.85 Bulletin 85/22**

(84) Etats contractants désignés: **AT BE CH DE FR GB IT LI LU NL SE**

(56) Documents cités:
**EP - A - 0 043 861**
**DE - A - 2 451 037**
**DE - C - 876 690**
**US - A - 2 974 172**
**US - A - 4 207 266**

(73) Titulaire: **RHONE-POULENC SPECIALITES CHIMIQUES, "Les Miroirs" 18, Avenue d'Alsace, F-92400 Courbevoie (FR)**

(72) Inventeur: **Desbois, Michel, 526, Chemin du Bois, F-69140-Rillieux (FR)**

(74) Mandataire: **Cazes, Jean-Marie et al, RHONE-POULENC RECHERCHES Service Brevets Chimie et Polymères 25, quai Paul Doumer, F-92408 Courbevoie Cedex (FR)**

**EP 0 092 627 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

**Description**

La présente invention a pour objet un procédé de préparation de di ou trifluorométhoxyphénylcétones ou di ou trifluorométhylthiophénylcétones.

On connait dans l'art antérieur des documents décrivant la préparation de composés de ce type.

On peut citer, notamment, la demande de brevet français publiée sous le numéro 2 272 079 qui décrit en particulier la préparation de l'isopropyl p-trifluorométhoxyphénylcétone selon laquelle on prépare un réactif de Grignard à partir d'oxyde de p-bromophényle et de trifluorométhyle et de magnésium. On le fait réagir avec de d'isobutyronitrile à reflux. Le produit est hydrolysé pour donner la cétone recherchée. On peut encore citer la demande de brevet français publiée sous le numéro 2 194 422 qui décrit un procédé analogue.

Ce type de procédé présente de sérieux inconvénients qui ne le rendent pas attractif au plan industriel. En effet, les étapes sont nombreuses et les temps de réaction sont longs. De plus, les rendements sont médiocres puisqu'ils n'atteignent que 30 à 40%. D'autre part, et cela n'est surement pas le moins important, la mise en oeuvre d'un organomagnésium et des solvants qui l'accompagnent est dangereuse pour l'environnement: il est nécessaire de prendre des précautions au plan technique ce qui alourdit l'économie du procédé.

On connait encore dans l'art antérieur des documents (voir par exemple OLAH, »Friedel-Crafts and Related Reactions — III — Part I — 1964 — Interscience Publishers« pages 8 et suivantes) qui décrivent l'acylation de substrats aromatiques (autres que les trifluorométhoxybenzènes ou trifluorométhylthiobenzènes) en présence de catalyseurs de Friedel et Crafts comme $AlCl_3$.

Les expériences de la demanderesse ont montré que les catalyseurs de Friedel et Crafts comme $AlCl_3$ sont inefficaces lorsque le composé aromatique de départ comporte un groupement $OCF_3$ ou $SCF_3$. Ces derniers sont même dégradés.

En présence d'acide sulfurique, autre catalyseur classique, les groupements $OCF_3$ et $SCF_3$ sont de même dégradés.

La demanderesse a maintenant découvert un nouveau procédé permettant d'obtenir des di ou trifluorométhoxyphénylcétones ou de di ou trifluorométhylthiophénylcétones à partir des di ou trihalogénométhoxybenzènes ou di ou trihalogénométhylthiobenzènes correspondants, ce qui n'avait pu être obtenu dans l'art antérieur.

La présente invention a pour objet un procédé de préparation de di ou trihalogénométhoxyphénylcétones ou di ou trifluorométhylthiophénylcétones caractérisé en ce que, dans une première étape, on fait réagir un die ou trihalogénométhoxybenzène ou un di ou trihalogénométhylthiobenzène ayant pour formule générale:

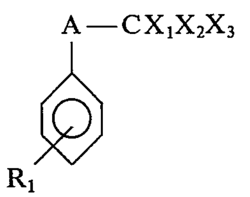

(I)

dans laquelle $X_1$, et $X_2$ identiques ou différents représentent Cl, Br, I ou F, $X_3$ représente H, Cl, Br, I ou F, A représente O ou S et $R_1$ représente au moins un élément choisi parmi le groupe comprenant l'hydrogène et les radicaux alkyle et alkoxy ayant de 1 à 6 atomes de carbone, les radicaux phényle et phénoxy substitués par au moins un groupement plus désactivant que le groupement $A-CX_1X_2X_3$, OH, Cl, Br, I et F avec un composé aromatique ou aliphatique trihalogénométhylé en présence de trifluorure de bore en quantité telle que la pression absolue de trifluorure de bore dans l'enceinte réactionnelle soit supérieure à 1 bar et en présence d'acide fluorhydrique comme solvant et en ce que, dans une deuxième étape, on hydrolyse le produit résultant.

On entend au sens de la présente invention par di ou trihalogénométhoxy benzène et di ou trihalogénométhylthiobenzène d'une part, ces produits proprement dits et, d'autre part, leurs homologues correspondant à la présence d'un ou plusieurs radicaux substituant le noyau benzénique.

Les radicaux $R_1$ phényle et phénoxy doivent être substitués par des groupements plus désactivants que le groupement $A-CX_1X_2X_3$ pour que la réaction se fasse sur le noyau benzénique porteur du groupement $A-CX_1X_2X_3$. Dans le cas contraire la réaction se ferait sur le radical phényle ou phénoxy. On peut citer comme exemples de groupements plus désactivants que le groupement $A-CX_1X_2X_3$, les groupements COOH, CN, $NO_2$, $CX_1X_2X_3$ et cétones.

Les composés de formule I dans laquelle $X_1$, $X_2$ et $X_3$ sont identiques sont encore plus particulièrement concernés par la présente invention. On préfère mettre en oeuvre parmi ceux-ci les composés dans lesquels $X_1$, $X_2$ et $X_3$ représentent le fluor.

On peut citer comme exemples de tels composés, les composés suivants: le trifluorométhoxybenzène, le trifluorométhylthiobenzène, l'o-, m- et p-chlorotrifluorométhoxybenzène, l'o-, m-, et p-chlorotrifluorométhylthiobenzène, l'o-, m- et p-bromotrifluorométhylthiobenzène, l'o-, m- et p-bromotrifluorométhoxybenzène, l'o-, m- et p-méthyltrifluorométhoxybenzène, l'o-, m- et p-méthyltrifluorométhyl-

thiobenzène, l'o-, m- et p-méthoxytrifluorométhoxybenzène, l'o-, m- et p-méthoxytrifluorométhylthiobenzène, l'o-, m- et p-hydroxytrifluorométhoxybenzène, l'o-, m- et p-hydroxytrifluorométhylthiobenzène, le trifluorométhyl-4 trifluorométhoxy-4' biphényle, le nitro-3 trifluorométhoxy-4' diphényle oxyde (on peut également citer les homologues chlorés, bromés ou iodés des composés ci-dessus) $\alpha,\alpha$-difluorométhoxybenzène, $\alpha,\alpha$-difluorométhylthiobenzène, le difluorobromométhoxybenzène, le difluorobromométhylthiobenzène, le dichlorofluorométhoxybenzène, le dichlorofluorométhylthiobenzène, le difluorochlorométhoxybenzène, le difluorochlorométhylthiobenzène.

On entend, au sens de la présente invention, par composé aromatique ou aliphatique trihalogénométhylé, un composé de formule:

$$R_2-CX_4X_5X_6 \qquad (II)$$

dans laquelle:
$R_2$ représente un radical aliphatique ou aromatique et $X_4X_5$ et $X_6$ identiques ou différents représentent Cl, Br ou F.

L'invention est particulièrement bien adaptée à la mise en oeuvre d'un composé de formule II dans laquelle $R_2$ représente un radical alkyle, phényle, alkylphényle, phénylalkyle ou phényle comportant au moins un substituant comme, par exemple, un halogène, $NO_2$, CN, $NH_2$, COOH.

Selon un mode de réalisation particulier de l'invention, $X_4$, $X_5$ et $X_6$ sont identiques. Encore plus particulièrement, on mettra en oeuvre un composé de formule II dans laquelle $X_4$, $X_5$ et $X_6$ représentent Cl.

On peut citer comme exemples de composés II, les composés suivants: trichloro-1,1,1 éthane, trichlorométhylbenzène, trifluorométhylbenzène, parafluorotrichlorométhylbenzène, parachlorotrifluorométhylbenzène, parachlorotrichlorométhylbenzène, orthochlorotrichlorométhylbenzène, métanitrotrichlorométhylbenzène, dichloro-3,4 trichlorométhylblenzène.

La première étape est mise en oeuvre, de préference, en utilisant kune quantité d'acide fluorhydrique telle que le rapport molaire de l'acide fluorhydrique au di ou trihalogénométhoxybenzène ou au di ou trihalogénométhylthiobenzène est compris entre 5 et 50. Encore plus préférentiellement, ce rapport est compris entre 10 et 30.

L'acide fluorhydrique mis en oeuvre est de préference anhydre. La mise en oeuvre d'acide fluorhydrique aqueux entrainerait une consommatioin inutile de trifluorure de bore sous forme de HF, $BF_3$, $H_2O$ ($H_3O^+BF_4^-$).

Le di ou trihalogénométhoxybenzène et le di ou trihalogénométhylthiobenzène et le composé de formule II sont utilisés en quantité sensiblement équimolaires. Un excès du secon peut cependant être souhaitable afin de minimiser la formation des composés de polycondensation.

On préfère plus particulièrement utiliser une quantité de trifluorure de bore telle que la pression absolue initiale de $BF_3$ dans l'enceinte réactionnelle soit comprise entre 6 et 20 bars. Plus la pression sera élevée, plus la vitesse de réaction augmentera. Une pression supérieure à 20 bars n'est pas exclue du domaine de l'invention, mais n'apporte pas d'avantage particulier. L'homme de l'art adaptera donc la pression à l'économie du procédé.

Lorsque l'on met en oeurvre comme composé I ou II un dérivé trichlorométhylé, on observe une élévation de la pression due à l'échange Cl—F.

La première étape est de préférence mise en oeuvre à une température comprise entre —20° C et 150° C.

Les temps de réaction sont généralement compris entre quelques minutes et plusieurs heures.

La deuxième étape est une hydrolyse qui peut être conduite en milieu acide ou basique comme cela est classique.

Une manière pratique de mettre en oeuvre le procédé selon l'invention est d'effectuer l'hydrolyse sur le mélange brut ou partiellement débarrassé du solvant HF issu de la première étape. On sera donc en présence d'HF donc en milieu acide.

L'élimination totale de l'HF antérieurement à la deuxième étape permet d'opérer soit en milieu basique soit en milieu acide.

L'hydrolyse se fait de préférence, à une température comprise entre 0 et 80° C.

Le procédé selon l'invention peut être schématisé de la façon suivante:

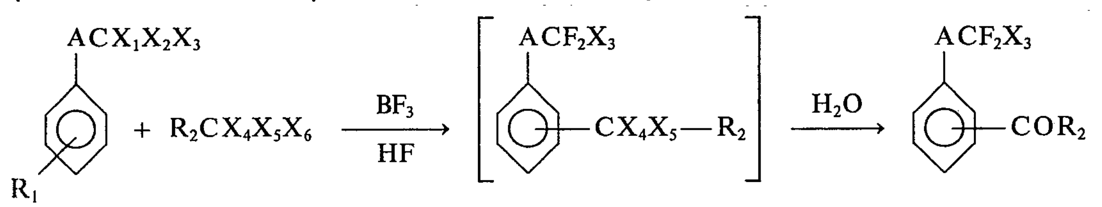


Quand $X_3$ représente un halogène, lors de la première étape qui s'effectue en milieu HF, les groupements $ACCl_3$, $ACBr_3$, $ACl_3$, $ACF_2Br$, $ACCl_2F$, $ACF_2Cl$ etc. se transforment en $A-CF_3$.

Quand $X_3$ représente un H, il ne s'échange pas, le groupement obtenu étant $ACF_2H$.

La position du groupement $-COR_2$ par rapport aux groupements $A-CF_2X_3$ et $R_1$ obéit aux règles de substitution bien connues du chimiste organicien.

Les cétones obtenues par le procédé de l'invention sont utiles, notamment, comme intermédiaires de synthèse de composés ayant une activité pharmaceutique ou phytosanitaire.

On peut citer comme exemples de composés pouvant être préparés par le procédé selon l'invention: trifluorométhoxy-4 chloro-2' benzophénone, trifluorométhoxy-4 chloro-4'benzophénone, trifluorométhoxy-4 benzophénone, trifluorométhoxy-4 acétophénone, trifluorométhoxy-4 fluoro-4' benzophénone, trifluorométhoxy-4 trifluorométhyl-4' benzophénone, trifluorométhoxy-4 trifluorométhyl-2' benzophénone, trifluorométhoxy-4 chloro-2 nitro-4' benzophénone, trifluorométhoxy-2 méthyl-5 amino-4' benzophénone, trifluorométhoxy-2 chloro-4 fluoro-4' benzophénone, trifluorométhoxy-3 hydroxy-4 cyano-3' benzophénone, $\alpha,\alpha$-difluorométhoxy-4 fluoro-4' benzophénone, trifluorométhoxy-3 dichloro-4',6 benzophénone, trifluorométhoxy-2 dichloro-4'5 benzophénone.

On peut également citer les composés homologues trifluorométhylthio.

L'invention va être maintenant plus complètement décrite à l'aide des exemples qui vont suivre. Ceux-ci ne sauraient être considérés comme limitant de façon quelconque l'invention.

## Exemple 1

Dans un réacteur inox de 250 ml muni d'un système d'agitation magnétique, on introduit aux environs de 0°C, 100 ml d'HF anhydre, 42,7 g (0,2 mole) de p-fluorotrichlorométhylbenzène et 32,4 g (0,2 mole) de trifluorométhoxybenzène.

Le réacteur est fermé puis on introduit du trifluorure de bore gazeux jusqu'à la pression constante de 6 bars. On laisse alors réagir sous agitation pendant 3 heures à la température ambiante. Après réaction, on décomprime le rèacteur jusqu'à pression atmosphérique puis on coule le mélange réactionnel sur 200 g de glace pilée. Après réchauffement à température ambiante, le mélange hétérogène ainsi obtenu est agité pendant une à deux heures, puis extrait par trois fois 200 $cm^3$ de chlorure de méthylène. Les phases organiques sont lavées par trois fois 200 $cm^3$ d'eau, une fois 200 $cm^3$ d'une solution aqueuse à 3% de potasse et deux fois 200 $cm^3$ d'une solution aqueuse à 3% de potasse et deux fois 200 $cm^3$ d'eau. La phase organique ainsi traitée est séchée sur sulfate de magnésium et le solvant est éliminé par distillation sous pression réduite. On recueille ainsi 51,9 g (rendement: 76,6%) de trifluorométhoxy-4 fluoro-4' benzophénone brute.

## Exemple 2

On procède comme à l'exemple 1 avec les composés et conditions suivants:

| | |
|---|---|
| — Acide fluorhydrique anhydre: | 100 g |
| — Trichloro-1,1,1 éthane: | 40,5 g 0,3 mole |
| — Trifluorométhkoxybenzène: | 16,2 g 0,1 mole |
| — Trifluorure de bore: | 6 bars a 20°C |
| — Température: | 10°C |
| — Durée: | 5 heures |

On recueille 16 g (rendement: 78,4%) de p-trifluorométhoxy-acétophénone brute.

## Exemple 3

On procède comme à l'exemple 1 avec les composés et conditions suivants:

| | |
|---|---|
| — Acide fluorhydrique anhydre: | 100 g |
| — p-chlorotrichlorométhylbenzène: | 46 g 0,2 mole |
| — Trichlorométhoxybenzène: | 42,3 g 0,2 mole |
| — Trifluorure de bore: | 6 bars à 20°C |
| — Température: | 95°C |
| — Durée: | 5 heures |

On recueille 47,6 g (rendement: 67%) de trifluorométhoxy-4 chloro-4' benzophénone brute.

Exemple 4

On procède comme à l'exemple 1 avec les composés et conditions suivants:

- Acide fluorhydrique anhydre: 100 g
- Trichlorométhylbenzène: 19,6 g 0,1 mole
- Trifluorométhoxybenzène: 16,2 g 0,1 mole
- Trifluorure de bore: 6 bars à 20° C
- Température: 40° C
- Durée: 4 heures

On recueille 26 g (rendement: 97,7%) de trifluorométhoxy-4 benzophénone brute.

Exemple 5

On procède comme à l'exemple 1 avec les composés et conditions suivants:

- Acide fluorhydrique anhydre: 100 g
- Trichlorométhylbenzène: 29,3 g 0,15 mole
- Difluorobromométhoxybenzène: 33,5 g 0,15 mole
- Trifluorure de bore: 8 bars à 20° C
- Température: 80° C
- Durée: 4 heures

On recueille 28,3 g (rendement: 71%) de trifluorométhoxy-4 benzophénone brute.

Exemple 6

On procède comme à l'exemple 1 avec les composés et conditions suivants:

- Acide fluorhydrique anhydre: 100 g
- p-chlorotrichlorométhylbenzène: 46 g 0,2 mole
- Trifluorométhylthiobenzène: 35,6 g 0,2 mole
- Trifluorure de bore: 10 bars a 20° C
- Température: 40° C
- Durée: 8 heures

On recueille 42,7 g (rendement: 67,5%) de trifluorométhylthio-4 chloro-4′ benzophénone brute.

Exemple 7

On procède comme à l'exemple 1 avec les composés et conditions suivants:

- Acide fluorhydrique anhydre: 100 g
- p-chlorotrifluorométhoxybenzène: 19,7 g 0,1 mole
- p-chlorotrichlorométhylbenzène: 23 g 0,1 mole
- Trifluorure de bore: 6 bars à 20° C
- Température: 120° C
- Durée: 18 heures

On recueille 7,4 g (rendement: 22%) d'un mélange brut de trifluorométhoxy-3 dichloro-4′6 benzophénone et de trifluorométhoxy-2 dichloro-4′, 5 benzophénone.

Exemple 8

On procède comme à l'exemple 1 avec les composés et conditions suivants:

- Acide fluorhydrique anhydre: 100 g
- α,α-difluorométhoxybenzène: 28,8 g 0,2 mole
- p-fluorotrichlorométhylbenzène: 42,7 g 0,2 mole
- Trifluorure de bore: 10 bars a 20° C
- Température: 50° C
- Durée: 3 heures

On recueille 38,2 g (rendement: 71%) de difluorométhoxy-4, fluoro-4′ benzophénone brute.

**Revendications**

1. Procédé de préparation de di ou trifluorométhoxyphénylcétones ou di ou trifluorométhylthiophénylcétones caractérisé en ce que dans une première étape, on fait réagir un di ou trihalogénométhoxybenzène ou un di ou trihalogénométhylthiobenzène ayant pour formule:

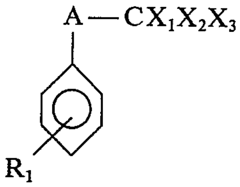

(I)

dans laquelle $X_1$ et $X_2$ identiques ou différents représentent Cl, Br, I ou F, $X_3$ représente H, Cl, Br, I ou F, A représente O ou S et $R_1$ représente au moins un élément choisi parmi-le groupe comprenant l'hydrogène et les radicaux alkyle et alkoxy ayant de 1 à 6 atomes de carbone environ, les radicaux phényle et phénoxy substitués par au moins un groupement plus désactivant que le groupement $A-CX_1X_2X_3$, OH, Cl, Br, I et F avec un composé aromatique ou aliphatique trihalogénométhylé en présence de trifluorure de bore en quantité telle que la pression absolue de trifluorure de bore dans l'enceinte réactionnelle soit supérieure à 1 bar et en présence d'acide fluorhydrique comme solvant et en ce que dans une deuxième étape, on hydrolyse le produit résultant.

2. Procédé selon la revendication 1 caractérisé en ce que dans la formule I, $X_1$, $X_2$ et $X_3$ sont identiques.

3. Procédé selon la revendication 2 caractérisé en ce que $X_1$, $X_2$ et $X_3$ représentent le fluor.

4. Procédé selon l'une quelconque des revendications précédentes caractérisé en ce que le composé aromatique ou aliphatique trihalogénométhylé répond à la formule:

$$R_2CX_4X_5X_6 \quad \text{(II)}$$

où $R_2$ représente un reste aliphatique ou aromatique et où $X_4$, $X_5$ et $X_6$ identiques ou différents représentent Cl, Br ou F.

5. Procédé selon la revendication 4 caractérisé en ce que dans la formule II, $R_2$ représente un radical alkyle, phényle, alkylphényle, phénylalkyle ou phényle comportant au moins un substituant halogène, $NO_2$, CN, $NH_2$, COOH.

6. Procédé selon l'une quelconque des revendications précédentes caractérisé en ce qu'on utilise une quantité d'acide fluorhydrique telle que le rapport molaire de l'acide fluorhydrique au di ou trihalogénométhoxybenzène ou au die ou trihalogénométhylthiobenzène est compris entre 5 et 50.

7. Procédé selon l'une quelconque des revendications précédentes caractérisé en ce que l'acide fluorhydrique mis en oeuvre est de l'acide fluorhydrique anhydre.

8. Procédé selon l'une quelconque des revendications précédentes caractérisé en ce que le di ou trihalogénométhoxybenzène ou di ou trihalogénométhylthiobenzène et le composé de formule II sont utilisés en quantité sensiblement équimolaires.

9. Procédé selon l'une quelconque des revendications précédentes caractérisé en ce qu'on utilise une quantité de trifluorure de bore telle que la pression absolue de $BF_3$ dans l'enceinte réactionnelle soit comprise entre 6 et 20 bars.

10. Procédé selon l'une quelconque des revendications précédentes caractérisé en ce que, dans la première étape, on opère à une température comprise entre $-20°$ C et 150° C.

11. Procédé selon l'une quelconque des revendications précédentes caractérisé en ce que, dans la deuxième étape, on opère à une température comprise entre 0 et 80° C et en milieu acide ou basique.

**Patentansprüche**

1. Verfahren zur Herstellung von Di- oder Trifluormethoxyphenylketonen oder Di- oder Trifluormethylthiophenylketonen, dadurch gekennzeichnet, daß man in einer ersten Stufe ein Di- oder Trihalogenmethoxybenzol oder ein Di- oder Trihalogenmethylthiobenzol mit der Formel:

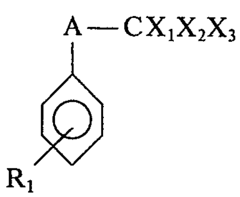

(I)

in der $X_1$ und $X_2$, die gleich oder verschieden sein können, Cl, Br, I oder F, $X_3$ H, Cl, Br, I oder F bedeuten,

A O oder S ist und $R_1$ wenigstens ein Element enthält, das aus der Gruppe ausgewählt ist, die Wasserstoff, die Alkyl- und Alkoxyradikale mit 1 bis 6 Kohlenstoffatomen, die mit wenigstens einer stärker desaktivierenden Gruppe als der Gruppe $A-CX_1X_2X_3$, OH, Cl, Br, I und F substituierten Phenyl- und Phenoxyradikale umfaßt, mit einer aromatischen oder aliphatischen Trihalogenmethylverbindung in Gegenwart von Bortrifluorid in einer derartigen Menge, daß der absolute Druck des Bortrifluorids im Reaktionsraum über 1 bar beträgt, und in Gegenwart von Fluorwasserstoffsäure als Lösungsmittel umsetzt und daß man in einer zweiten Stufe das erhaltene Produkt hydrolysiert.

2. Verfahren nach Anspruch 1, dadurch gekennzeichnet, daß in der Formel I $X_1$, $X_2$ und $X_3$ identisch sind.

3. Verfahren nach Anspruch 2, dadurch gekennzeichnet, daß $X_1$, $X_2$ und $X_3$ Fluor sind.

4. Verfahren nach einem der vorangehenden Ansprüche, dadurch gekennzeichnet, daß die aromatische oder aliphatische Trihalogenmethylverbindung die Formel besitzt:

$$R_2CX_4X_5X_6 \qquad (II)$$

in der $R_2$ einen aliphatischen oder aromatischen Rest bedeutet und $X_4$, $X_5$ und $X_6$, gleich oder verschieden, Cl, Br oder F bedeuten.

5. Verfahren nach Anspruch 4, dadurch gekennzeichnet, daß in Formel II $R_2$ ein Alkyl-, Phenyl-, Alkylphenyl-, Phenylalkyl- oder Phenylradikal mit wenigstens einem Halogen-, $NO_2$-, CN-, $NH_2$- oder COOH-Substituenten ist.

6. Verfahren nach einem der vorangehenden Ansprüche, dadurch gekennzeichnet, daß man eine derartige Menge Fluorwasserstoffsäure einsetzt, daß das Molverhältnis zwischen Fluorwasserstoffsäure und dem Di- oder Trihalogenmethoxybenzol oder dem Di- oder Trihalogenmethylthiobenzol zwischen 5 und 50 liegt.

7. Verfahren nach einem der vorangehenden Ansprüche, dadurch gekennzeichnet, daß die eingesetzte Fluorwasserstoffsäure wasserfreie Fluorwasserstoffsäure ist.

8. Verfahren nach einem der vorangehenden Ansprüche, dadurch gekennzeichnet, daß das Di- oder Trihalogenmethoxybenzol oder Di- oder Trihalogenmethylthiobenzol und die Verbindung der Formel II in ungefähr äquimolarer Menge eingesetzt werden.

9. Verfahren nach einem der vorangehenden Ansprüche, dadurch gekennzeichnet, daß man eine derartige Menge Bortrifluorid einsetzt, daß der absolute Druck des $BF_3$ im Reaktionsraum zwischen 6 und 20 bar liegt.

10. Verfahren nach einem der vorangehenden Ansprüche, dadurch gekennzeichnet, daß man in der ersten Stufe bei einer Temperatur zwischen −20°C und 150°C arbeitet.

11. Verfahren nach einem der vorangehenden Ansprüche, dadurch gekennzeichnet, daß man in der zweiten Stufe bei einer Temperatur zwischen 0 und 80°C in saurem oder basischem Milieu arbeitet.

**Claims**

1. Process for preparing di- or trifluoromethoxyphenyl ketones or di- or trifluoromethylthiophenyl ketones, characterised in that, in a first stage, a di- or trihalomethoxybenzene or a di- or trihalomethylthiobenzene having the formula:

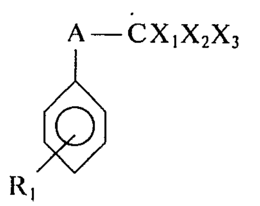

(I)

in which $X_1$ and $X_2$, which are identical or different, denote Cl, Br, I or F, $X_3$ denotes H, Cl, Br, I or F, A denotes O or S and $R_1$ denotes at least one member chosen from the group comprising hydrogen and the alkyl and alkoxy radicals containing from 1 to approximately 6 carbon atoms, the phenyl and phenoxy radicals substituted by at least one group which is more deactivating than the group $A-CX_1X_2X_3$, OH, Cl, Br, I and F, is reacted with an aromatic or aliphatic trihalomethyl compound in the presence of boron trifluoride in such quantity that the absolute pressure of boron trifluoride in the reaction enclosure is greater than 1 bar and in the presence of hydrofluoric acid as a solvent, and in that, in a second stage, the resulting product is hydrolysed.

2. Process according to Claim 1, characterised in that, in the formula I, $X_1$, $X_2$ and $X_3$ identical.

3. Process according to Claim 2, characterised in that $X_1$, $X_2$ and $X_3$ denote fluorine.

4. Process according to any one of the preceding claims, characterised in that the aromatic or aliphatic trihalomethyl compound corresponds to the formula:

$$R_2CX_4X_5X_6 \qquad (II)$$

where $R_2$ denotes an aliphatic or aromatic residue and where $X_4$, $X_5$ and $X_6$, which are identical or different, denote Cl, Br or F.

5. Process according to Claim 4, characterised in that, in the formula II, $R_2$ denotes an alkyl, phenyl, alkylphenyl, or phenylalkyl radical, or a phenyl radical containing at least one substituent halogen, $NO_2$, CN, $NH_2$, or COOH.

6. Process according to any one of the preceding claims, characterised in that a quantity of hydrofluoric acid is employed such that the molar ratio of hydrofluoric acid to the di- or trihalomethoxybenzene or to the di- or trihalomethylthiobenzene is between 5 and 50.

7. Process according to any one of the preceding claims, characterised in that the hydrofluoric acid employed is anhydrous hydrofluoric acid.

8. Process according to any one of the preceding claims, characterised in that the di- or trihalomethoxybenzene or di- or trihalomethylthiobenzene and the compound of formula II are employed in substantially equimolar quantities.

9. Process according to any one of the preceding claims, characterised in that a quantity of boron trifluoride is employed such that the absolute pressure of $BF_3$ in the reaction enclosure is between 6 and 20 bars.

10. Process according to any one of the preceding claims, characterised in that, in the first stage, it is operated at a temperature of between $-20°C$ and $150°C$.

11. Process according to any one of the preceding claims, characterised in that, in the second stage, it is operated at a temperature of between 0 and 80°C and in an acidic or basic medium.